# EUROPÄISCHE PATENTSCHRIFT

(11) **EP 2 007 293 B1**
(45) Veröffentlichungstag und Bekanntmachung des Hinweises auf die Patenterteilung: **23.01.2013**
(21) Anmeldenummer: 07723942.4
(22) Anmeldetag: 03.04.2007
(51) Int. Cl.: A61B 17/3203, A61B 18/14, A61B 18/12

(54) **ENDOSKOPISCHES MULTIFUNKTIONS-CHIRURGIEGERÄT**
MULTI-FUNCTION DEVICE FOR ENDOSCOPIC SURGERY
INSTRUMENT CHIRURGICAL MULTIFONCTIONS ENDOSCOPIQUE

(30) Priorität: 11.04.2006 DE 102006017014; 16.06.2006 DE 102006027873
(43) Veröffentlichungstag der Anmeldung: 31.12.2008
(73) Patentinhaber: ERBE Elektromedizin GmbH, 72072 Tübingen (DE)
(72) Erfinder: FISCHER, Klaus, 72202 Nagold (DE)
(74) Vertreter: Bohnenberger, Johannes
(86) Internationale Anmeldenummer: PCT/EP2007/003001
(87) Internationale Veröffentlichungsnummer: WO 2007/118608

(56) Entgegenhaltungen:
- EP-A- 0 280 972
- EP-A2- 1 790 299
- DE-A1- 3 715 418
- DE-A1- 4 420 608
- DE-A1-102004 013 419
- DE-A1-102004 020 855

## Beschreibung

Die Erfindung betrifft ein endoskopisches Multifunktions-Chirurgiegerät, insbesondere für die Wasserstrahlchirurgie und HF-chirurgische Anwendungen nach dem Oberbegriff der Patentansprüche 1 und 2.

Minimal-invasive Chirurgie bezeichnet als Oberbegriff operative Eingriffe mit kleinstem Trauma. Schon immer war es Ziel der operativen Behandlung, eine rasche Genesung mit geringen Beschwerden nach der Operation einzuleiten. Zu Beginn der 90er Jahre des vergangenen Jahrhunderts etablierte sich die laparoskopische und endoskopische Chirurgie zunächst nur mit einfachen operativen Eingriffen, später jedoch auch zur Durchführung komplexerer Operationen.

Dabei wird heute zwischen der laparoskopischen Chirurgie und der Endoskopie unterschieden. In der laparoskopischen Chirurgie werden so zusagen die gleichen Eingriffe gemacht, wie mit den offenen chirurgischen Methoden. Der größte Unterschied zu den herkömmlichen Eingriffen liegt allerdings darin, daß man das Operationsgebiet durch wesentlich kleinere Schnitte erreicht, als mit den offenen Methoden.

Demgegenüber kann in der Endoskopie ein Arzt ohne einen großen chirurgischen Eingriff gut in die natürlichen Körperhöhlen und Hohlorgane des Patienten blicken, Krankheiten erkennen und sie gegebenenfalls auch sofort behandeln. Dazu werden flexible oder starre Endoskope zur Untersuchung der Organe und z.B. zum Betrachten derer Schleimhaut eingesetzt. Zu diesem Zweck gibt es auch Endoskope mit unterschiedlichen Außendurchmessern, Längen, Biopsiekanaldurchmessern und Funktionen. Außerdem wird die so genannte interventionelle Endoskopie heute nicht mehr ausschließlich in der Diagnostik eingesetzt, sondern häufig auch in der Therapie der verschiedensten Krankheiten.

Typische Anwendungen für die laparoskopische Chirurgie und/oder die interventionelle Endoskopie sind z.B. die selektive Gewebetrennung durch Hochfrequenz- (HF) oder Wasserstrahlchirurgie und die Blutstillung (Koagulation) oder Gefäßversiegelung durch HF-Zangen oder Elektroden. Überdies werden chirurgische Zangen auch zur Dissektion oder Biopsieentnahme von Gewebe, oder auch nur zur Gewebe-Präparation oder Fixierung verwendet.

Die endoskopische Mukosaresektion (EMR), bei der großflächige Tumore im Gastrointestinaltrakt entfernt werden, wird z.B. mit Hilfe der Wasserstrahltechnik durchgeführt, wobei zur Blutstillung (Koagulation) die HF-Chirurgie eingesetzt wird. Dabei werden die Blutgefäße, die zuvor mit der Wasserstrahltechnik selektiv vom Gewebe getrennt wurden, gezielt mit HF-Zangen sicher verschlossen.

In der Wasserstrahlchirurgie wird dabei ein hauchfeiner laminarer Wasserstrahl genutzt, der das Gewebe sozusagen auseinanderdrängt und einen Expansionsraum bildet. Weiche Gewebe können grundsätzlich bei einem geringen Druck disseziert werden, wobei Gewebe mit hoher Elastizität oder großer Dehnung, wie z.B. Blutgefäße, dem Wasserstrahl ausweichen und dadurch geschont werden.

Demgegenüber wird in der HF-Chirurgie elektrische Energie in Wärme umgewandelt und ist so in der Lage, biologisches Gewebe zu trennen oder auch eine Blutung zu stillen. Dabei werden hauptsächlich thermische Effekte genutzt. Temperaturen von 60 bis 70°C im Bereich um die HF-Chirurgie-Elektrode führen zur Eiweißgerinnung. Man spricht von der Koagulation. Mit diesem "Verschweißungseffekt" können z.B. Blutungen zum Stillstand gebracht werden.

Beim Trennen werden durch eine höhere Stromdichte Temperaturen von über 100°C erreicht, so dass die Flüssigkeit explosionsartig verdampft, der Raum vergrößert wird und die Zellmembran sozusagen platzt. Weitere, in Richtung der Elektrodenbewegung liegende Zellen folgen diesem Effekt, wodurch der gewünschte Schnitt oder Trennung des Gewebes erreicht wird.

In der HF-Chirurgie unterscheidet man außerdem zwischen der monopolaren und der bipolaren Anwendungstechnik. Bei der monopolaren Anwendungstechnik erfolgt der Stromfluss von einer HF-Chirurgie-Elektrode durch das biologische Gewebe zu einer Neutralelektrode, welche meistens großflächig am Patienten platziert ist. Im Gegensatz dazu fließt bei der bipolaren Anwendungstechnik der HF-Strom nicht über den Körper des Patienten zu einer Neutralelektrode. Bei bipolaren Pinzetten, Zangen oder Klemmen werden eine Aktivelektrode und eine Neutralelektrode direkt einander gegenüber angeordnet, wobei der HF-Strom nur von der Aktivelektrode zur Neutralelektrode fließt. Somit ergeben sich sehr kurze Stromwege und umgrenzte Koagulationsbezirke mit geringem Leistungsbedarf.

Während einer Operation z.B. EMR sind häufig verschiedene Instrumente zum Greifen, Spülen, Trennen und/oder Koagulieren von Gewebe notwendig und müssen entsprechend in den Arbeitskanälen ausgetauscht werden. Der ständige Austausch der Instrumente erfordert jedoch viel Zeit und kann den Verlauf einer Operation erheblich verlängern.

Eine Multifunktions-Vorrichtungen, die z.B. ein HF Handinstrument zum bipolaren Koagulieren, Schneiden und Greifen sowie ein in einem Schutzrohr zusätzlich untergebrachtes Spülrohr und Absaugrohr umfasst, ist aus der DE 42 42 143 C2 bekannt. Die zusätzlich in einem Schutzrohr untergebrachten Rohre (Spülen, Absaugen) erlauben jedoch kein selektives Trennen durch HF- und Wasserstrahlchirurgie und benötigen unter anderem zusätzlichen Raum, da sie parallel zu den Elektroden an das Handinstrument angebracht sind.

Des Weiteren beschreibt die DE 100 56 238 A1 eine Vorrichtung mit einer Maulmechanik für Rohrschaftinstrumente zum Entnehmen intrakorporaler Gewebeproben, welche durch einen Rohrschaft in proximale Richtung abgesaugt werden können. Der geöffnete Mauldurchgang kann zusätzlich zum Spülen oder Absaugen oder auch zum Einführen von Koagulationselektroden, Optiken oder anderen Arbeitssonden genutzt werden. Allerdings müssen die genutzten Instrumente und Sonden während des Eingriffs entsprechend ausgewechselt werden.

Die Aufgabe der Erfindung besteht nun darin, ein Endoskopisches Multifunktions-Chirurgiegerät zu schaffen, welches universell verwendbar ist, und dabei wenig Platz in Anspruch nimmt.

Diese Aufgabe wird durch ein Gerät nach Patentansprüche 1 und 2 gelöst.

Insbesondere wird die Aufgabe durch ein endoskopisches Multifunktions-Chirurgiegerät mit einer Zufuhreinrichtung zur Zufuhr mindestens einer Flüssigkeit und einer Zange oder Klemme die zangenförmige Elektroden mit Maulteilen zur HF-Chirurgie umfasst gelöst, wobei die Zufuhreinrichtung zur Dissektion von Gewebe mittels Flüssigkeitsstrahl an oder in einem Maulteil ausgebildet ist.

Ein wesentlicher Punkt der Erfindung liegt darin, dass eine Vorrichtung zur Wasserstrahlchirurgie und HF-Elektroden zur Koagulation und/oder zum Schneiden so in oder an einer Zange oder Klemme ausgebildet sind, dass das Multifunktions-Chirurgiegerät nicht mehr Raum in Anspruch nimmt als ein herkömmliches HF-Instrument oder eine herkömmliche Zange oder Klemme.

Dadurch wird ein Multifunktions-Chirurgiegerät bereitgestellt, das die Vorteile der Wasserstrahl-Chirurgie, insbesondere die Dissektion mittels Flüssigkeitsstrahl, mit den Vorteilen der HF-Chirurgie, insbesondere HF-Schneiden und thermische Koagulation, in einer Zange oder Klemme, die nicht größer ist als eine herkömmliches HF-Instrument, verbindet.

Der Vorteil des erfindungsgemäßen Multifunktionsgerätes besteht somit insbesondere darin, dass mit Hilfe eines einzigen Multifunktions-Chirurgiegerätes die Funktionen des selektiven Schneidens, des Fassens und des thermischen Koagulierens von Gewebe durchgeführt werden können, ohne dass ein Instrumentenwechsel während des Eingriffs erforderlich ist. Folglich können Operationszeiten, Kosten und auch die Risiken für den Patienten bzgl. der Eingriffsdauer minimiert werden.

Das endoskopische Multifunktions-Chirurgiegerät umfasst zangenförmige Elektroden mit einem starren und einem beweglichen Maulteil, wobei die Zufuhreinrichtung zu Dissektion mittels Flüssigkeitsstrahl an oder in dem starren Maulteil ausgebildet ist. Das starre Maulteil bietet hierbei eine einfache Möglichkeit, die Zufuhreinrichtung in einem der Maulteile zu integrieren und anwendungsgemäß zu kontrollieren.

Weiterhin umfasst das die Zufuhreinrichtung beinhaltende Maulteil ein Endstück, das aus dem distalen Ende des Maulteils heraussteht, wobei das Endstück der Zufuhreinrichtung als monopolare Elektrode ausgebildet ist, die zur Dissektion und/oder Koagulation von Gewebe geeignet ist. Das Endstück der Zufuhreinrichtung fungiert somit nicht nur als monopolare HF-Elektrode zur Dissektion von Gewebe sonder auch als Austrittdüse für ein präziseres Scheiden durch die Wasserstrahl-Chirurgie.

Vorzugsweise umfasst die monopolare Elektrode am distalen Ende des Endstücks der Zufuhreinrichtung eine insbesondere kreisförmig ausgebildete Scheibe und/oder einen insbesondere halbkugelförmigen Aufsatz umfasst, welche das HF-Schneiden in alle Richtungen vereinfacht.

Weiterhin ist das Endstück bezüglich einer Längsachse des starren Maulteils bewegbar. Dadurch wird erreicht, dass die Länge der Elektrode entsprechend dem Einsatz angepasst werden kann.

In einer anderen Ausführungsform der Erfindung ist mindestens ein Ausgang der Zufuhreinrichtung an der dem beweglichen Maulteil gegenüberliegenden Innenseite des starren Maulteils so angeordnet, dass mindestens ein Flüssigkeitsstrahl in Richtung des beweglichen Maulteils ausstoßbar ist. Dadurch wird erreicht, dass auf einfache Weise eine präzisere Gewebetrennung bewirkt werden kann, wie diese z.B. bei der Leberteilresektion vorteilhaft ist. Das Gewebe kann gleichzeitig durch die Maulteile fixiert werden. Außerdem bietet das dem Ausgang der Zufuhreinrichtung gegenüberliegenden Maulteil unter anderem Schutz gegen den mit sehr hohem Druck austretenden mindestens einen Flüssigkeitsstrahl. Dabei ist der mindestens eine Ausgang der Zufuhreinrichtung bezüglich einer Längsachse des starren Maulteils verschiebbar. Dadurch wird erreicht, dass der Flüssigkeitsstrahl gegenüber dem durch die beiden Maulteile fixierten Gewebe verschoben werden kann und die Gewebetrennung somit erleichtert wird.

Des Weiteren befindet sich der mindestens eine Ausgang der Zufuhreinrichtung in einer zu einer Längsachse des Multifunktions-Chirurgiegerätes parallel angeordneten Vertiefung des starren Maulteils und/oder das beweglichen Maulteil umfasst eine zur Längsachse des Multifunktions-Chirurgiegeräts parallel angeordnete Vertiefung, die dem mindestens einen Ausgang der Zufuhreinrichtung gegenüber liegt. Dadurch wird erreicht, dass der mindestens eine Ausgang beim Trennen durch den Flüssigkeitsstrahl und zusätzlichen Fixieren des zu trennenden Gewebes freigehalten wird und die austretende Flüssigkeit über die Vertiefungen abgeleitet werden kann.

Eine weitere Ausführungsform der Erfindung sieht vor, dass die Zange oder Klemme als Biopsiezange ausgebildet ist, so dass das Multifunktions-Chirurgiegerät insbesondere zur Biopsieentnahme eingesetzt werden kann.

Weiterhin ist es möglich, dass die Maulteile des erfindungsgemäßen Multifunktions-Chirurgiegerätes durch ein Federelement vorgespannt und dadurch offen gehalten werden. Dadurch wird insbesondere die Funktion des Greifens und Koagulierens erleichtert, da die Maulteile nur noch aktiv geschlossen werden müssen, sich aber durch das Federelement wieder selbständig öffnen.

Weitere Ausführungsformen der Erfindung ergeben sich aus den Unteransprüchen.

Nachfolgend wird die Erfindung anhand von Ausführungsbeispielen beschrieben, die anhand der Abbildungen näher erläutert werden. Hierbei zeigen:
- - Fig. 1: eine vereinfachte Darstellung eines typischen Einsatz einer Ausführungsform eines Multifunktions-Chirurgiegerätes, wobei ein Blutgefäß durch den Flüssigkeitsstrahl frei präpariert und nachfolgend getrennt und koaguliert werden kann;
- - Fig. 2: eine perspektivische Darstellung einer Ausführungsform eines erfindungsgemäßen Multifunktions-Chirurgiegerätes;
- - Fig. 3: eine perspektivische Darstellung einer weiteren Ausführungsform eines erfindungsgemäßen Multifunktions-Chirurgiegerätes mit fester Zufuhreinrichtung;
- - Fig. 4: eine perspektivische Darstellung einer weiteren Ausführungsform eines erfindungsgemäßen Multifunktions-Chirurgiegerätes mit beweglicher Zufuhreinrichtung;
- - Fig. 5: eine Schnittdarstellung entlang der Linie V-V in Fig. 3;
- - Fig. 6: eine Seitenansicht einer weiteren Ausführungsform eines erfindungsgemäßen Multifunktions-Chirurgiegerätes, dass als Biopsiezange ausgebildet ist, und
- - Fig. 7: eine Seitenansicht einer weiteren Ausführungsform eines erfindungsgemäßen Multifunktions-Chirurgiegerätes, wobei das starre Maulteil als funktioneller Schnitt dargestellt ist.

In der nachfolgenden Beschreibung werden für gleiche und gleich wirkende Teile dieselben Bezugsziffern verwendet.

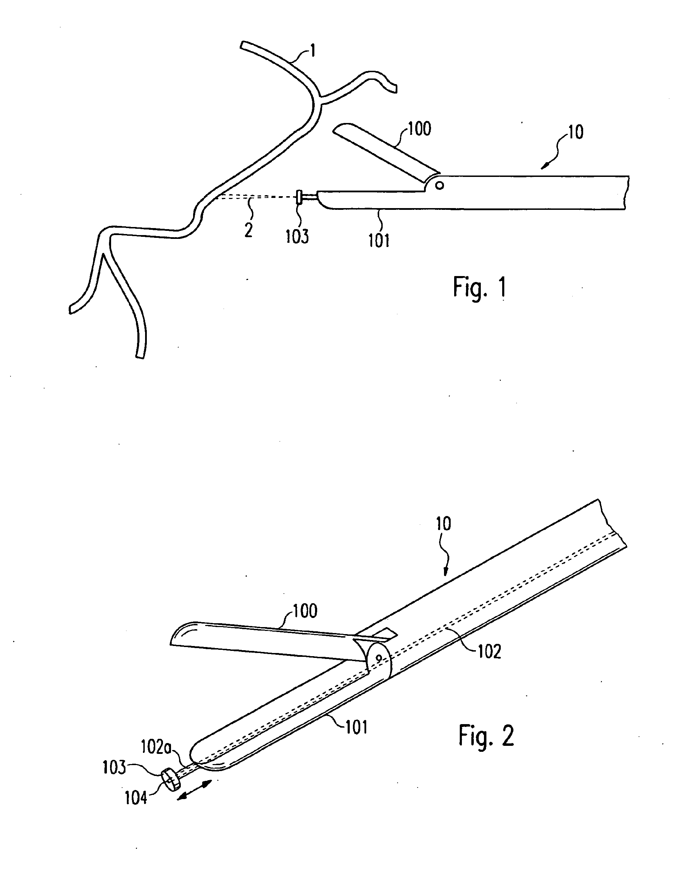

In dem in Figur 1 und Figur 2 dargestellten Ausführungsbeispiel ist ein Multifunktions-Chirurgiegerät 10 mit einer Zange oder Klemme mit jeweils einem beweglichen Maulteil 100 und einem starren Maulteil 101 gezeigt. Die Maulteile 100, 101 sind zugleich als bipolare Elektroden ausgebildet. Eine kreisscheibenförmig ausgebildete monopolare Elektrode 103 ist an einem Endstück 102a einer in dem starren Maulteil 101 integrierten und in Figur 2 nicht sichtbaren Zufuhreinrichtung 102 zu Bildung eines Flüssigkeitsstrahls 2 für eine Flüssigkeit, insbesondere NaCl-Lösung, angebracht. Das Multifunktions-Chirurgiegerät ist dabei so ausgebildet, dass es z.B. Gewebe um ein Blutgefäß 1 selektiv dissektieren kann, ohne das Blutgefäß zu beschädigen. Nach dem Freipräparieren wird das Blutgefäß 1 mit den bipolaren Elektroden 100, 101 der Zange oder Klemme getrennt und durch Koagulation zu "verschweißt". Die als Elektroden ausgebildete Maulteile 100, 101 werden dabei durch einen höheren Stromfluss zwischen den bipolaren Elektroden 100, 101 und den daraus resultierenden höheren Temperaturen zur Gewebetrennung eingesetzt. Zusätzlich erlauben die als Zange oder Klemme ausgebildeten Maulteile 100, 101 ein Greifen oder Fassen und ermöglichen somit z.B. die Präparation des zu operierenden Gewebes, Organs oder Blutgefäßes. Alle soeben genannten Funktionen stehen mit dem erfindungsgemäßen Multifunktions-Chirurgiegerät ohne Instrumentenwechsel zur Verfügung, was wiederum die Operationsdauer beträchtlich verkürzen kann und folglich Risiken für den Patienten minimiert. Des weiteren können die Zange oder Klemme in einer weiteren vorteilhaften Ausführung nach außen hin elektrisch isoliert sein.

Ferner kann die Einrichtung zur Zufuhr einer Flüssigkeit 102 mit einem entsprechend geringeren Druck des Flüssigkeitsstrahls auch zum Injizieren oder Spülen genutzt werden. Je nach Anwendungsgebiet kann die HF- Elektrode 103 als Nadel, Haken, Spatel, Halbkugel, Scheibe oder in jeglicher anderen vorteilhaften Form ausgebildet sein. Außerdem kann die HF-Elektrode 103 derart in das starre Maulteil 101 integriert sein, dass sie bezüglich einer Längsachse des starren Maulteils bewegbar ist. Weiterhin kann das Multifunktions-Chirurgiegerät 10 als starres oder flexibles Instrument ausgebildet sein.

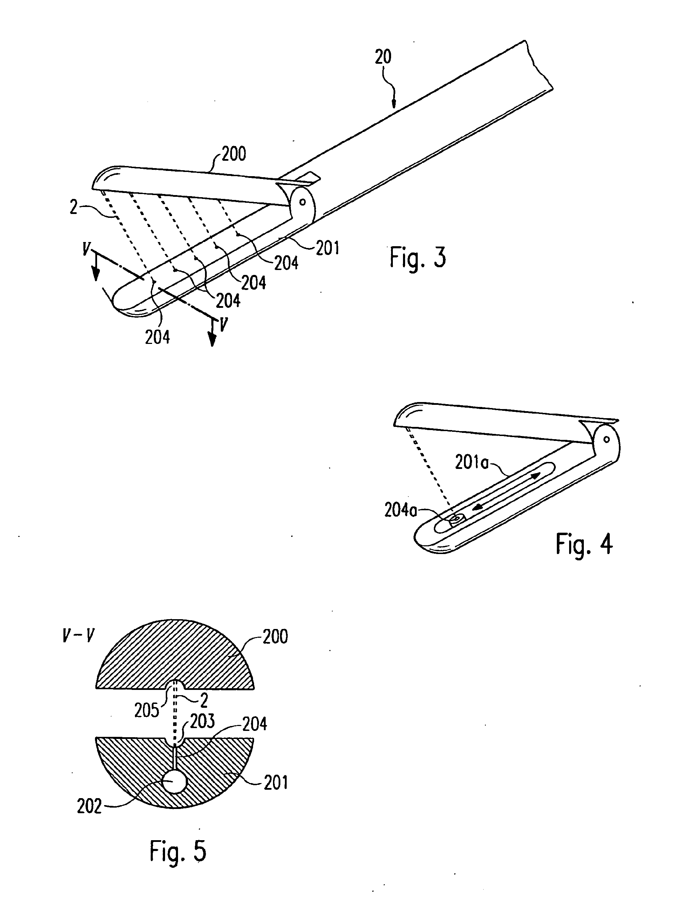

Ein weiteres Ausführungsbeispiel der Erfindung ist in den Figuren 3, 4 und 5 dargestellt. Dieses Ausführungsbeispiel ist besonders für präzises Trennen von Gewebe wie z.B. bei Leberteilresektionen geeignet. Hierbei ist mindestens ein Ausgang 204 einer Zufuhreinrichtung 202 für eine Flüssigkeit an einer dem beweglichen Maulteil 200 gegenüberliegenden Innenseite des starren Maulteils 201 angeordnet, so dass mindestens ein Flüssigkeitsstrahl 2 in Richtung des beweglichen Maulteils 200 mit geeignetem Druck ausstoßbar ist. Dabei wird das geöffnete Multifunktions-Chirurgiegerät 20 mit einer Schiebebewegung in Richtung des Gewebes bewegt und das Gewebe wird durch mindestens einen Flüssigkeitsstrahl getrennt. Die Ausgänge 204 sind dabei in einer Vertiefung 203 des starren Maulteils 201 angeordnet, um eine Blockierung durch das Gewebe zu verhindern. Weiterhin kann das bewegliche Maulteil 200 auch als Schutz für Gewebe in unmittelbarer Nähe gegen die mit hohem Druck austretenden Flüssigkeitsstrahlen 2 wirken und eine Perforation anstehender Gewebeteile verhindern. Der auf das bewegliche Maulteil auftreffende Flüssigkeitsstrahl wird außerdem durch die Vertiefung 205 abgeleitet. Fig. 4, zeigt hierbei eine Ausführungsform mit einem bezüglich einer Längsachse des starren Maulteils 102a verschiebbaren Ausgang 204a.

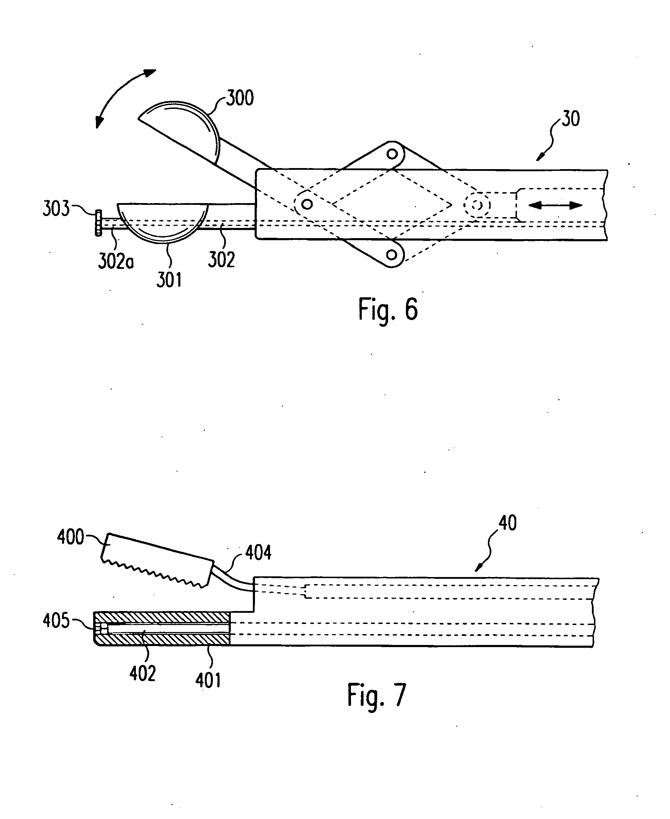

Das in Figur 6 dargestellte Ausführungsbeispiel zeigt ein Multifunktions-Chirurgiegerät 30 mit einer speziell für die Biopsie ausgebildeten Biopsiezange, welche ein starres Maulteil 301 und ein bewegliches Maulteil 300 umfasst. In dem starren Maulteil 301 ist eine Zufuhreinrichtung 302 für einen Flüssigkeitsstrahl 2 einer Flüssigkeit, insbesondere NaCl-Lösung integriert. Das Endstück 302a der Zufuhreinrichtung 302 tritt hierbei aus dem starren Maulteil 302 hervor, wobei eine kreisscheibenförmige monopolare Elektrode 303 am Ende des Endstücks 302a ausgebildet ist. Die monopolare Elektrode am Ende des Endstücks 302a kann natürlich auch jede andere zum HF-Schneiden vorteilhafte Form haben. Die Funktion der an sich bekannten Greifmechanik der Biopsiezange ist dabei durch Pfeile gekennzeichnet. Die verdeckten Teile der Greifmechanik sind durch gestrichelte Linien dargestellt. Das in Figur 6 dargestellte erfindungsgemäße Ausführungsbeispiel hat den Vorteil, dass Gewebeteile selektiv durch HF- oder Wasserstrahlchirurgie abgetrennt werden können und danach gleich mit den Maulteilen 300, 301 der Biopsiezange 30 aufgenommen und abtransportiert werden können. Bei geringeren Stromstärken könnte die monopolare Elektrode 303 auch zum Koagulieren eingesetzt werden.

Ein weiteres Ausführungsbeispiel der Erfindung ist in der Figur 7 dargestellt. Hierbei ist das bewegliche Maulteil 400 durch ein Federelement 404 derartig vorgespannt, dass es offen gehalten wird. Außerdem wird in Figur 7 ein Schnitt durch ein starres Maulteil dargestellt, wobei eine in das starre Maulteil integrierte Zufuhreinrichtung 402 für einen Flüssigkeitsstrahl 2 und auch eine am Ende der Zufuhreinrichtung 402 ausgebildete Düse 405 sichtbar sind. Das starre und das bewegliche Maulteil 400, 401 sind als bipolare Elektroden für die Koagulation von Gewebe ausgebildet und können bei entsprechender Stromstärke auch als HF-Schneidelektroden verwendet werden.

### Bezugszeichenliste

- 1: Blutgefäß
- 2: Flüssigkeitsstrahl

- 10: erste Ausführungsform eines Multifunktions-Chirurgiegerätes
- 100: bewegliches Maulteil
- 101: starres Maulteil
- 102: Zufuhreinrichtung eines Flüssigkeitsstrahls
- 102a: Endstück der Zufuhreinrichtung
- 103: HF-Elektrode in z.B. Scheibenform
- 104: Ausgang der Zufuhreinrichtung

- 20: zweite Ausführungsform eines Multifunktions-Chirurgiegerätes
- 200: bewegliches Maulteil
- 201: starres Maulteil
- 201 a: starres Maulteil für verschiebbaren Ausgang der Zufuhreinrichtung
- 202: Zufuhreinrichtung eines Flüssigkeitsstrahls
- 203: Vertiefung des starren Maulteils
- 204: Ausgang der Zufuhreinrichtung
- 204a: Verschiebbarer Ausgang der Zufuhreinrichtung
- 205: Vertiefung des beweglichen Maulteils

- 30: dritte Ausführungsform eines Multifunktions-Chirurgiegerätes
- 300: bewegliches Maulteil
- 301: starres Maulteil
- 302: Zufuhreinrichtung eines Flüssigkeitsstrahls
- 302a: Endstück der Zufuhreinrichtung
- 303: HF-Elektrode in z.B. Scheibenform

- 40: vierte Ausführungsform eines Multifunktions-Chirurgiegerätes
- 400: bewegliches Maulteil
- 401: starres Maulteil
- 402: Zufuhreinrichtung eines Flüssigkeitsstrahls
- 405: Düse

## Patentansprüche

1. Endoskopisches Multifunktions-Chirurgiegerät mit einer Zufuhreinrichtung (102) zur Zufuhr mindestens einer Flüssigkeit und mit einer Zange oder Klemme, die zangenförmige Elektroden mit Maulteilen (100, 101) zur HF-Chirurgie umfasst,
**dadurch gekennzeichnet, dass**
die zangenförmigen Elektroden ein starres (101) und ein bewegliches (100) Maulteil umfassen und die Zufuhreinrichtung (102) zur Dissektion von Gewebe mittels Flüssigkeitsstrahl an oder in dem starren Maulteil (101) ausgebildet ist, wobei ein Endstück (102a) der Zufuhreinrichtung (102) aus dem distalen Ende des starren Maulteils (101) heraussteht und als HF-Elektrode ausgebildet ist, die zur Dissektion und/oder Koagulation von Gewebe geeignet ist, wobei das Endstück (102a) bezüglich einer Längsachse des starren Maulteils (101) bewegbar ist derart, dass die Länge der HF-Elektrode entsprechend dem Einsatz angepasst werden kann.

2. Endoskopisches Multifunktions-Chirurgiegerät mit einer Zufuhreinrichtung (202) zur Zufuhr mindestens einer Flüssigkeit und mit einer Zange oder Klemme, die zangenförmige Elektroden mit Maulteilen (200, 201) zur HF-Chirurgie umfasst,
**dadurch gekennzeichnet, dass**
die Zufuhreinrichtung (202) zur Dissektion von Gewebe mittels Flüssigkeitsstrahl an oder in einem der Maulteile (200, 201) ausgebildet ist, wobei die zangenförmigen Elektroden ein starres (201) und ein bewegliches (200) Maulteil umfassen, wobei die Zufuhreinrichtung (202) an oder in dem starren Maulteil (201) ausgebildet ist,
wobei mindestens ein Ausgang (204) der Zufuhreinrichtung (202) an der dem beweglichen Maulteil (200) gegenüberliegenden Innenseite des starren Maulteils (201) so angeordnet ist, dass mindestens ein Flüssigkeitsstrahl (2) in Richtung des beweglichen Maulteils (200) ausstoßbar ist und der mindestens eine Ausgang (204a) der Zufuhreinrichtung (202) bezüglich einer Längsachse des starren Maulteils (201) derart verschiebbar ist dass der Flüssigkeitsstrahl gegenüber dem durch die beiden Maulteile (200, 201) fixierten Gewebe verschoben werden kann und die Gewebetrennung somit erleichtert wird.

3. Multifunktions-Chirurgiegerät nach Anspruch 1,
**dadurch gekennzeichnet, dass**
die HF-Elektrode am distalen Ende des Endstücks (102a) der Zufuhreinrichtung (102) eine kreisförmig ausgebildete Scheibe (103) und/oder einen halbkugelförmigen Aufsatz umfasst.

4. Multifunktions-Chirurgiegerät nach Anspruch 2,
**dadurch gekennzeichnet, dass**
sich der mindestens eine Ausgang (204, 204a) der Zufuhreinrichtung (202) in einer zu einer Längsachse des Multifunktions-Chirurgiegerätes (20) parallel angeordneten Vertiefung (203) des starren Maulteils (201) befindet und/oder das bewegliche Maulteil (200) eine zur Längsachse des Multifunktions-Chirurgiegerätes (20) parallel angeordnete Vertiefung (205) umfasst, die dem mindestens einen Ausgang (204) der Zufuhreinrichtung (202) gegenüber liegt.

5. Multifunktions-Chirurgiegerät nach einem der vorhergehenden Ansprüche, insbesondere nach Anspruch 2,
**dadurch gekennzeichnet, dass**
die Zange oder Klemme als Biopsiezange ausgebildet ist.

6. Multifunktions-Chirurgiegerät nach einem der vorhergehenden Ansprüche, insbesondere nach Anspruch 2,
**dadurch gekennzeichnet, dass**
die Maulteile (100, 400) durch ein Federelement (404) vorgespannt sind und dadurch offen gehalten werden.

## Claims

1. Multi-function device for endoscopic surgery, with a delivery means (102) for delivering at least one liquid, and with a forceps or clamp that comprises forceps-shaped electrodes with jaw parts (100, 101) for HF surgery, **characterized in that** the forceps-shaped electrodes comprise a rigid jaw part (101) and a movable jaw part (100), and the delivery means (102) for dissection of tissue by means of a jet of liquid is formed on or in the rigid jaw part (101), wherein an endpiece (102a) of the delivery means (102) protrudes from the distal end of the rigid jaw part (101) and is designed as an HF electrode suitable for the dissection and/or coagulation of tissue, wherein the endpiece (102a) is movable relative to a longitudinal axis of the rigid jaw part (101), in such a way that the length of the HF electrode can be adapted according to the use.

2. Multi-function device for endoscopic surgery, with a delivery means (202) for delivering at least one liquid, and with a forceps or clamp that comprises forceps-shaped electrodes with jaw parts (200, 201) for HF surgery, **characterized in that** the delivery means (202) for dissection of tissue by means of a jet of liquid is formed on or in one of the jaw parts (200, 201), wherein the forceps-shaped electrodes comprise a rigid jaw part (201) and a movable jaw part (200), wherein the delivery means (202) is formed on or in the rigid jaw part (201), wherein at least one outlet (204) of the delivery means (202) is arranged on the inside face of the rigid jaw part (201) opposite the movable jaw part (200), such that at least one liquid jet (2) can be ejected in the direction of the movable jaw part (200), and the at least one outlet (204a) of the delivery means (202) is movable relative to a longitudinal axis of the rigid jaw part (201), in such a way that the liquid jet can be moved relative to the tissue fixed by the two jaw parts (200, 201), and tissue separation is thus made easier.

3. Multifunction device for surgery according to Claim 1, **characterized in that** the HF electrode at the distal end of the endpiece (102a) of the delivery means (102) comprises a circular disc (103) and/or a hemispherical attachment.

4. Multifunction device for surgery according to Claim 2, **characterized in that** the at least one outlet (204, 204a) of the delivery means (202) is located in a recess (203) of the rigid jaw part (201) arranged parallel to a longitudinal axis of the multifunction device for surgery (20) and/or the movable jaw part (200) comprises a recess (205) which is arranged parallel to the longitudinal axis of the multifunction device for surgery (20) and lies opposite the at least one outlet (204) of the delivery means (202).

5. Multifunction device for surgery according to one of the preceding claims, in particular according to Claim 2, **characterized in that** the forceps or clamp is designed as a biopsy forceps.

6. Multifunction device for surgery according to one of the preceding claims, in particular according to Claim 2, **characterized in that** the jaw parts (100, 400) are pretensioned by a spring element (404) and thus held open.

## Revendications

1. Appareil endoscopique chirurgical multifonction doté d'un dispositif d'amenée (102) qui amène au moins un liquide et d'une pince ou tenaille qui comporte des électrodes en forme de pince et dotées de pièces de mâchoires (100, 101) pour la chirurgie HF,
**caractérisé en ce que**
les électrodes en forme de pince comprennent une partie immobile de mâchoire (101) et une partie mobile de mâchoire (100),
**en ce que** le dispositif d'amenée (102) est configuré pour disséquer un tissu à l'aide d'un jet de liquide sur ou dans la partie immobile de mâchoire (101),
**en ce qu'**une pièce d'extrémité (102a) du dispositif d'amenée (102) déborde de l'extrémité distale de la partie immobile de mâchoire (101) et est configurée comme électrode HF qui convient pour la dissection et/ou la coagulation de tissus et
**en ce que** la pièce d'extrémité (102a) peut être déplacée par rapport à l'axe longitudinal de la partie immobile de mâchoire (101) de telle sorte que la longueur de l'électrode HF puisse être adaptée aux besoins de l'utilisation.

2. Appareil endoscopique chirurgical multifonction doté d'un dispositif d'amenée (202) qui amène au moins un liquide et d'une pince ou tenaille qui comporte des électrodes en forme de pince et dotées de pièces de mâchoires (200, 201) pour la chirurgie HF,
**caractérisé en ce que**
le dispositif d'amenée (202) est formé sur ou dans l'une des parties de mâchoires (200, 201) pour la dissection de tissus au moyen d'un jet de liquide,
**en ce que** les électrodes en forme de pince comprennent une partie immobile de mâchoire (201) et une partie mobile de mâchoire (200),
**en ce que** le dispositif d'amenée (202) est formé sur ou dans la partie immobile de mâchoire (201),
**en ce qu'**au moins une sortie (204) du dispositif d'amenée (202) est disposée sur le côté intérieur de la partie immobile de mâchoire (201), opposé à la partie mobile de mâchoire (200), de telle sorte qu'au moins un jet de liquide (2) puisse être expulsé en direction de la partie mobile de mâchoire (200) et
**en ce que** la ou les sorties (204a) du dispositif d'amenée (202) peuvent coulisser par rapport à l'axe longitudinal de la partie immobile de mâchoire (201) de telle sorte que le jet de liquide puisse être déplacé par rapport au tissu fixé par les deux parties de mâchoire (200, 201) pour ainsi faciliter la séparation du tissu.

3. Appareil chirurgical multifonction selon la revendication 1, **caractérisé en ce que** l'électrode HF comprend une plaque (103) de forme circulaire et/ou un appendice de forme hémisphérique à l'extrémité distale de la pièce d'extrémité (102a) du dispositif d'amenée (102).

4. Appareil chirurgical multifonction selon la revendication 2, **caractérisé en ce que** la ou les sorties (204, 204a) du dispositif d'amenée (202) sont situées dans un creux (203) de la partie immobile de mâchoire (201) disposé parallèlement à l'axe longitudinal de l'appareil chirurgical multifonction (20) et/ou **en ce que** la partie mobile de mâchoire (200) comprend un creux (205) disposé parallèlement à l'axe longitudinal de l'appareil chirurgical multifonction (20) et situé face à la ou les sorties (204) du dispositif d'amenée (202).

5. Appareil chirurgical multifonction selon l'une des revendications précédentes, en particulier selon la revendication 2, **caractérisé en ce que** la pince ou tenaille est configurée comme pince de biopsie.

6. Appareil chirurgical multifonction selon l'une des revendications précédentes, en particulier selon la revendication 2, **caractérisé en ce que** les parties de mâchoire (100, 400) sont précontraintes par un élément élastique (404) et sont ainsi maintenues ouvertes.
